# EUROPEAN PATENT APPLICATION

(11) **EP 1 795 587 A1**
(43) Date of publication of application: **13.06.2007**
(21) Application number: 05111806.5
(22) Date of filing: 07.12.2005
(51) Int. Cl.: C12N 5/06

(54) **Method for the direct culture of dendritic cells without a preceding centrifugation step**

(71) Applicant: Schuler, Gerold, Prof. Dr., 91080 Spardorf (AT)
(72) Inventor: Schuler, Gerold, Prof.Dr., 91080 Spardorf (DE); Erdmann, Michael Karl, 91080 Spardorf (DE); Schuler-Thurner, Beatrice, 91080 Spardorf (DE)
(74) Representative: Helbing, Jörg

(57) **Abstract**

The present invention provides a method for the direct culture of dendritic cell precursors enriched by elutriation without a preceding centrifugation step to generate dendritic cells.

## Description

The present invention provides a method for the direct culture of dendritic cell precursors enriched by elutriation without a preceding centrifugation step to generate dendritic cells.

### Background of the Invention

The adoptive transfer of antigen-loaded dendritic cells (DC), i.e. DC vaccination, is a promising approach to manipulate the immune system (reviewed in Banchereau J. and Palucka A. K., Nat. Rev. Immunol. 5(4):296-306 (2005) and Nestle F. O. et al., Curr. Opin. Immunol. 17(2): 163-9 (2005). In murine models mature DC ("immunostimulatory DC") have been used to induce immunity to cancer and infection by inducing effector T cells, and immature or semimature DC ("tolerogenic DC") were employed to induce antigen-specific tolerance by inducing regulatory T cells. In man more than 1000 cancer patients have been vaccinated with "immunostimulatory DC" in clinical trials. DC vaccination appeared particularly immunogenic, and in some clinical trials promising clinical effects have been observed. DC vaccination is, however still in an early stage of development. Many critical variables have not yet been addressed (e.g. DC type and maturation stimulus; dose, frequency, and route of injection etc.). A major problem is the fact that the generation of DC vaccines is still expensive and time consuming and at the same time not yet standardized, in part because a straightforward closed system is not yet available.

DC are most often generated from monocytes. To this end adherent fractions of peripheral blood mononuclear cells (isolated from freshly drawn blood or aphereses which are highly enriched in monocytes (Putz T. et al., Methods Mol. Med. 109:71-82 (2005)), or monocytes isolated from peripheral blood, e.g. by magnetic separation from apheresis products using the CliniMacs System (Berger T. G. et al., J. Immunol. 268(2):131-40 (2002); Campbell J. D. et al., Methods Mol. Med. 109:55-70 (2005); Babatz J. et al., J. Hematother. Stem Cell Res. 12(5):515-23 (2003) and Motta M. R. et al., Br. J. Haematol. 121(2):240-50 (2003))) are exposed to GM-CSF + IL-4 usually over about 6 days to yield immature DC, and finally matured by adding a maturation stimulus (most often IL-1 alpha + IL-6 + TNF alpha + PGE2). Instead of GM-CSF + IL-4 other cytokine combinations can be used such as GM-CSF + IL13, GM-CSF + IL-15, GM-CSF + IFN alpha etc. (reviewed in Banchereau J. and Palucka A. K., Nat. Rev. Immunol. 5(4):296-306 (2005)), and monocytes can even be converted to DC by the use of CpG oligonucleotides (Krutzik S. R., Nat. Med. 11(6):653-60 (2005)). Also, a variety of maturation stimuli are available (Gautier G. et al., J. Exp. Med. 2;201(9):1435-46 (2005); Napolitani G. et al., Nat. Immunol. 6(8):769-76. Epub 2005 Jul 3 (2005) and reviewed in Banchereau J. and Palucka A. K., Nat. Rev. Immunol. 5(4):296-306 (2005)).

To test these and other variables and to perform larger, randomized clinical trials it is of utmost importance to develop a cost-effective, reproducible method to generate DC in a closed system. Adherence-based closed systems (such as the AastromReplicell System for production of dendritic cells, Aastrom Biosciences, Inc., Ann Arbor, MI, USA) require a large volume of culture media causing high costs of production, and require as an additional preceding step the isolation of PBMC from apheresis products, for which no straightforward, clinically approved system is available. The alternative approach is to first isolate monocytes. This has been possible for some time by using superparamagnetic iron-dextran particles directly conjugated to CD14 antibody and the CliniMACS Cell Selection System (Miltenyi Biotec GmbH, Bergisch Gladbach, Germany, see Berger T. G. et al., J. Immunol. 268(2): 131-40 (2002); Campbell J. D. et al., Methods Mol. Med. 109:55-70 (2005); Babatz J. et al., J. Hematother. Stem Cell Res. 12(5):515-23 (2003) and Motta M. R. et al., Br. J. Haematol. 121(2):240-50 (2003)) which is approved for clinical use in Europe but not in the USA. The system yields essentially pure fractions of positively selected monocytes which are delivered from the magnetic columns into bags in CliniMACS PBS/EDTA buffer. Highly enriched fractions of monocytes can also be obtained by immunomagnetic depletion of contaminating of T, B and NK cells (Berger T. G. et al., J. Immunol. Methods 298(1-2):61-72 (2005)) using the Isolex 300I Magnetic cell selector.

We recently described an alternative, and in our view preferable approach to isolate monocytes for DC generation, namely elutriation employing an advice which became recently available and is approved for clinical use (Elutra Cell Separation System^{™}, Gambro AB, Stockholm, Sweden; see Berger T. G. et al., J. Immunol. Methods 298(1-2):61-72 (2005)). Obvious advantages are that the enriched monocytes are untouched, and that the use of costly and non-humanized antibodies for magnetic separation is avoided. A disadvantage shared by the magnetic separation and the elutriation procedures is the fact that the cell factions are obtained in a salt solution, which necessitates at least one concentration step, i.e. a centrifugation step, before the start of the cell culture as the monocytes have to be sedimented for removal of the salt solution and have to be suspended in a suitable culture medium. According to today's standards such centrifugation cannot be performed under sterile conditions, rather has to be performed in an open system, i.e. by opening the hitherto closed container/bag and taking the cell fractions out of the container/bag. Even in a GMP laboratory such taking out is critical. A proposed solution to this problem is to centrifuge the closed container/bag and to remove the supernatant by suction, which is, however, quite cumbersome. This problem does not only occur in case of isolating and cultivating monocytes, but is immanent whenever cells (e.g. lymphocytes), which were isolated either by magnetic separation or the elutriation process, have to be put in culture.

### Summary of the Invention

We found that the elutriation procedure magnetic or other separation strategies can be modified so as to avoid such a potentially non-sterile centrifugation step. The method established allows the *direct* culture of cells enriched by elutriation, magnetic or other separation strategies *without* preceding centrifugation step, either
- by supplementing the vessel (e.g. the container, bag or the like) containing the cells which were enriched in salt solutions, i.e. solutions not close to the final culture medium, with a small volume containing appropriate concentrations of all supplements required to yield a suitable and desired culture medium, or
- by performing the enrichment step in a medium close to or identical with the final culture medium
   Thus the present invention provides a method for the sterile culture of blood-derived cells or bone marrow-derived cells, which method comprises enriching the cells by a separation procedure not requiring a centrifugation step and
   (1) performing the enrichment or eluation step of the separation process with a medium close to or identical with the medium required for culture of the cells, and collecting the cells in a vessel under sterile conditions, or
   (2) performing the enrichment or eluation step of the separation process with a medium not suitable for culture of the cells, collecting the cells in a vessel and supplementing the enriched cell suspension in the vessel with an appropriate amount of a composition containing appropriate concentrations of all supplements required to yield a suitable culture medium.

The method further relates to cells cultured by the methods (1) and (2) defined above.

### Brief Description of the Figures

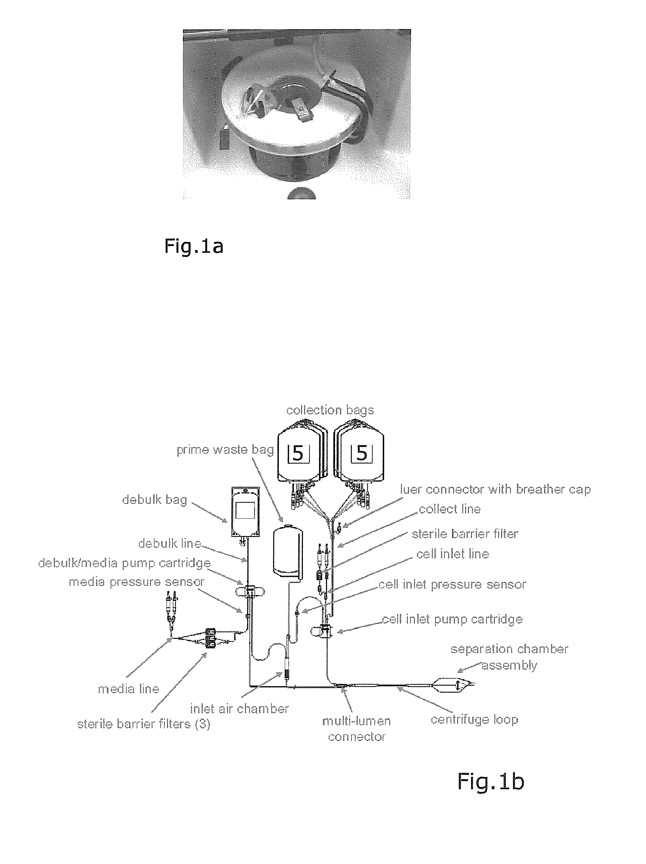

Fig. 1A: Elutra-cell separator: rotor containing the 40-ml elutriation chamber. B: Components of the disposable tubing set. The centrifuge loop consists of the following components: The multi-lumen tubing carries fluid in and out of the separation chamber assembly. The sleeves reinforce the tubing at the flex points. The collars connect the two ends of the loop in the centrifuge. The bearings provide contact points between the centrifuge arm and the loop. The debulk line carries RBCs and some granulocytes from the separation chamber to the debulk bag during RBC debulking. It also carries media solution during the Prime mode and debulk rinse. The debulk/media pump cartridge holds and organizes the debulk and media line tubing. The debulk bag (600 ml) stores red blood cells and some granulocytes removed during RBC debulking. It also stores media solution processed during Prime mode and debulk rinse. The collect line: during Prime mode, the collect line carries the media solution used during Prime mode to the prime collection bag. During Run mode, the collect line carries the collected components to a fraction collection bag. The collect line clamps open and close the lines to the prime collection bag, fraction collection bags, and the line with the blue breather cap. The collection bags (1 I) are manufactured from citrated PVC. The prime collection bag stores the media solution used during Prime mode. The fraction collection bags (5) store the fractions collected. The product sampler provides a means for taking a sample of the fraction collection bags. The prime waste bag stores the media solution used during Prime mode. The waste divert valve lines carry media solution to the prime waste bag during Prime mode.

### Detailled Description of the Invention

The present invention provides for an improved *ex-vivo* process for the culture of blood-derived cells under all-sterile conditions, namely a process meeting the GMP requirements. Cells to be cultured by the method of embodiments (1) and (2) of the invention include dendritic cells (DCs) and all other cells present in or derived from peripheral blood (or bone marrow) including stem cells and their progeny.

In a preferred embodiment the method (2) furthermore comprises a sterile cryopreservation step prior to the addition of the composition containing the supplements.

In the method (1) and (2) above, the separation process includes, but is not limited to, elutriation and magnetic separation. The elutriation is e.g. effected by utilizing the Elutra Cell Separation System^{™} (Gambro AB, Stockholm, Sweden) Berger T. G. et al., J. Immunol. Methods 298(1-2):61-72 (2005)). Magnetic separation is suitable e.g. for the isolation of monocytes from peripheral blood, and includes their magnetic separation from apheresis products using the CliniMacs System (Berger T. G. et al., J. Immunol. 268(2):131-40 (2002); Campbell J. D. et al., Methods Mol. Med. 109:55-70 (2005); Babatz J. et al., J. Hematother. Stem Cell Res. 12(5):515-23 (2003) and Motta M. R. et al., Br. J. Haematol. 121(2):240-50 (2003)). Both methods yield pure fractions of the desired cells such as monocytes, which are delivered into closed containers/bags. In the method of the invention the blood-derived cells or bone marrow-derived cells are preferably selected from monocytes, immature dendritic cells, CD34⁺ cells, stem cells, and any other cell contained therein.

Moreover it is preferred in the method of the invention that the vessel allows for sterile collection and culture of the cell suspension, sterile transfer to other culture or storage vessels, and optionally for sterile cryopreservation. It is preferred that the vessel is a plastic bag having sealable adapters, such as the Cell-Freeze bag type CF100-C3 containing a pre-attached DMSO filter, which allows for closed system cryopreservation.

The method of the invention may further comprise culture, differentiation and/or expansion of the cells under reaction conditions and by addition of supplements required for the culture, differentiation and/or expansion of the respective cells or precursor cells.

In a particular preferred embodiment of the invention, the method is suitable for the sterile culture of dendritic cell precursors to generate dendritic cells and comprises elutriation of peripheral blood mononuclear cells and culture of the monocytes obtained by the elutriation. The invention is hereinafter described by reference to said preferred embodiment, the generation of dendritic cells, which shall, however, not be construed to limit the invention.

In the method of the invention and in particular in a method for the generation of dendritic cells the preferred separation process is elutriation. The buffer for elutriation, i.e. the culture medium suitable for the elutriation and culture, includes, but is not limited to, RPMI medium supplemented with autologous plasma or serum or allogenic plasma or serum, preferably with about 1% of said plasma or serum (such as the commercially available RPMI 1640 medium of Bio Whittaker, Walkersville, USA, supplemented with 20 µg/ml gentamicin (Refobacin 10, Merck, Darmstadt, Germany), 2 mM glutamine (Bio Whit-taker), and 1% heat-inactivated (56 °C for 30 min) autologous human plasma), X-Vivo 15 medium (Cambrex Bio Science, Verviers, Belgium), CellGro DC Medium (Cell Genix GmbH Freiburg, Germany), AimV Culture Medium (Invitrogen, Carlsbad, CA, USA), and the like. Particularly preferred is the above-mentioned RPMI medium supplemented with 1% autologous human plasma or serum.

For the generation of dendritic cells the isolated monocytes are cultured while adding one or more differentiation factors such as cytokines and/or one or more maturation factors.

The differentiation factors include, but are not limited to, GM-CSF, IL-4, IL-13, IFN-α, IL-3, IFN-β, TNF-α, CPG oligonucleotides and combinations of said differentiation factors.

In particular, monocytes can be differentiated to stimulatory dendritic cells by the following differentiated factors and combinations of differentiated factors: GM-CSF and IL-4 (Romani, N. et al., J. Exp. Med. 180:83-93 (1994); Sallusto, F. and Lanzavecchia, A., J. Exp. Med. 179:1109-1118 (1994)), GM-CSF and IL-13 (Romani, N. et al., J. Immunol. Methods 196(2): 137-51 (1996)) GM-CSF + IFN-α (Carbonneil, C. et al., AIDS 17(12): 1731-40 (2003)), IL-3 and IL-4 (Ebner, S. et al., J. Immunol. 168(12):6199-207 (2002)), IL-3 and IFN-β (Buelens, C. et al., Blood 99(3):993-8 (2002)), IFN-α (Blanco, P. et al., Science 294:1540-1543 (2001); Luft, T. et al. J Immunol 161:1947-1953 (1998); Paquette, R. L. et al., J. Leukoc. Biol. 64:358-367 (1998); Santini, S. M. et al., J. Exp. Med. 191:1777-1788 (2000)). TNF-α (Chomarat, P. et al., J Immunol 171:2262-2269 (2003)), GM-CSF and IL-15 (Mohamadzadeh M. et al., J. Exp. Med. Oct 1;194(7):1013-20 (2001)), CpG oligonucleotides (primarily via induction of GM-CSF and other cytokines; Krutzik S.R. et al., Nat. Med. 11(6):653-60. (2005)). Particular preferred for the differentiation of stimulatory dendritic cells are a combination of GM-CSF and IL-4.

The present method is also applicable for the Generation of tolerogenic dendritic cells. Suitable conditions for the induction of such tolerogenic dendritic cells is e.g. described in the following articles the content of which is hereby incorporated in its entirety: Lutz M. B. and Schuler G., Trends Immunol. 23(9):445-9 (2002); Steinbrink K. et al., Blood 93(5):1634-42 (1999); Rea D. et al., Hum. Immunol. 65(11):1344-55 (2004); Bellinghausen I. et al., J. Allergy Clin. Immunol. 108(2):242-9 (2001); Jonuleit H. et al., J. Exp. Med. 192(9): 1213-22 (2000); Dhodapkar M.V. and Steinman R.M., Blood 100(1):174-7 (2002); Levings M. K. et al., Blood 105(3):1162-9 (2002); Rea D. et al., Blood 95(10):3162-7 (2000); and de Jong E. C. et al., J. Leukoc. Biol. 66(2):201-4. (1999).

A variety of maturation factors are available. Particular maturation factors and combination of maturation factors are the following: TNF-α, IL-1, IL-6 alone or in various combinations and optionally containing prostaglandins (monocyte conditioned medium: Thurner B. et al., J. Immunol. Methods 223(1):1-15 (1999); defined cocktail: Jonuleit H. et al., Eur. J. Immunol. 27(12):3135-42 (1997)), various TLR ligands (Napolitani G. et al., Nat. Immunol. 6(8):769-76 (2005); Gautier G. et al., J. Exp. Med. 201(9):1435-46 (2005)); CD40 crosslinking by CD40 ligand and optionally containing IFN-γ (Caux C. et al., J. Exp. Med. 180(4):1263-72 (1994); Koya R.C. et al., J. Immunother. 26(5):451-60 (2003)), type I interferon (Gautier G. et al., J. Exp. Med. 201(9):1435-46 (2005)) and combinations of the above maturation factors. Particularly preferred is a maturation cocktail comprising TNF-α, IL-1β, IL-6, and PGE₂.

Before, during or after the maturation the dendritic cells the dendritic cells or the respective dendritic cell precursors may be loaded with protein or lipid antigens, DNA or RNA coding for antigens, whole cells (preferably apoptotic or necrotic cells), cell fragments or cell lysates, or the like.

The method of the invention may further include one or more of the following steps: partitioning and/or cryopreservation of the cells cultured as defined hereinbefore.

As set forth above, the method of embodiments (1) and (2) of the invention corresponds to the following two solutions:
Solution I: This solution pertains to a process which comprises introducing into the vessels (viz. bags, containers or the like), which contain the desired cells in salt solution, all supplements required to convert the salt solution into a suitable culture medium. The all sterile conditions require, however, that the respective supplement(s) preparations are available in a quality approved for clinical use. Although this poses no principal problem such supplement(s) preparations in GMP or pharmaceutical preparation are not available at present.

Solution II: This second, preferred solution of the problem was to perform the elutriation in a culture medium which would be suitable for both the elutriation step and the subsequent culture of the desired cells. It was found that particular media such as the RPMI 1640 + 1% autologous plasma fulfilled the two requirements, and in addition is to the best of our knowledge the cheapest of the culture media approved for clinical use (see Example). It is important to note that this solution to the problem was not evident from prior art as elutriation (as well as magnetic separation) has generally been performed in salt solutions because of the possible interference of media components. The performance of elutriation - or, more generally spoken the isolation of cells - in media close to or identical with the final culture media, appears to be a small improvement but is in fact a big one from a practical point of view. By circumventing any centrifugation step after the monocyte enrichment and the initiation of culture a very simple functionally-closed cell separation system has become possible. In case of large scale DC production it merely requires three simple steps (which require only readily available instruments and reagents approved for clinical use), namely
i) apheresis resulting in a bag/container containing mononuclear cells,
ii) elutriation (or magnetic separation or other enrichment procedures) performed in media identical or close to the final culture media to yield the desired cells in a bag/container which contains the (final or next to final) culture medium, and
iii) injection into the bag(s)/container(s) of supplements (such as cytokines like GM-CSF and IL-4)
   to yield the desired final progeny (such as DC) upon culture of the cells enriched by elutriation (or magnetic separation).

The invention is further described by the following examples, which are, however, not to be construed as limiting the invention.

### Examples

### Example 1: Isolation of PBMC from healthy donors.

The leucapheresis was performed with healthy donors after informed consent was given as described in Strasser, E. F. et al., Transfusion 43(9): 1309 (2003). A modified MNC program was used for the Cobe Spectra (MNC program version 5.1) with an enhanced separation factor of 700. The inlet blood flow rate was 60 ml/min with a continuous collection rate of 0.8 ml/min. The anticoagulant was set to an ACD-A to blood ratio of 1:10 as basic adjustment for all procedures that was adjusted to a ratio of 1:11 or 1:12 in case of citrate reactions. The separation factor of 700 was equivalent to a centrifugation speed of 1184 rpm at a blood flow rate of 60 ml/min. The rate of the plasma pump was set manually by visual inspection of the cell interface within the separation chamber and the observation of the collection line to target a low hematocrit of approximately 1% to 2%.

### Example 2: Enrichment of monocytes by elutriation with culture medium.

Peripheral blood monocytes obtained in Example 1 were enriched directly from unmobilized leucapheresis products using an Elutrak cell separator (Gambro BCT, Lakewood, Colorado, USA) and single-use, functionally sealed disposable sets, containing a newly designed 40-ml elutriation chamber. It separates cells on the basis of sedimentation velocity, which is dependent on cell size and, to a lesser extent density. After prime, the leucapheresis product was dissolved in elutriation/culture medium (RPMI 1640 medium (Bio Whittaker, Walkersville, USA) supplemented with 20 µg/ml gentamicin (Refobacin 10, Merck, Darmstadt, Germany), 2 mM glutamine (Bio Whit-taker), and 1% heat-inactivated (56 °C for 30 min) autologous human plasma) and was then loaded into the elutriation chamber using the cell inlet pump and a centrifuge speed of 2400 rpm. Thereafter, the centrifuge speed was held constant, and the flow of elutriation/culture medium contained in two 3-I pooling bags (T3006, Cell-Max GmbH, Munich, Germany), was increased step-wise to allow for the elutriation of the specific cell fractions into the pre-attached collection bags (Fig. 1). The cell inlet pump speed was set at 37 ml/min, the media pump speed was gradually increased in 4 steps, namely 37-97.5-103.4-103.9 ml/min, and ~975 ml elutriation media per fraction. To collect the final fraction from the elutriation chamber (rotor off fraction), the centrifuge was stopped and the cells were pumped at 103.9 ml/min into the final collection bag. The total processing time was approximately one hour.

Cellular components of all collected fractions were manually counted by standard trypan blue dye exclusion. For performing flow cytometry, cells were labelled with anti-CD14-FITC and anti-CD45-CyCr mAbs (Becton Dickinson, New Jersey, USA) and analysed on a Cytomics FC500 (Beckman-Coulter, Miami, FL, USA) using RxP software. In addition, cells were studied in an automatic cell counter (Casy cell counter and analyzer system, model TT, Schärfe System, Reutlingen, Germany). This system uses pulse area analysis and allows for the objective measurement of cell numbers, cell size, and volume, as well as cell viability.

### Example 3: DC-generation

Monocytes obtained in Example 2 were separated into four culture bags CF100-C3 Cell-Freeze (Cell-Max GmbH, Munich, Germany; which is applicable for cryopreservation and contain tubings that allow for sterile docking) at a density of 1x10⁶/ml in 50-70 ml culture medium. under sterile conditions and were incubated at 37 °C and 5% CO₂. The bags were supplemented on days 1, 3, and 5 with rhu GM-CSF (final concentration 800 U/ml; Leukomax, Novartis, Nuremberg, Germany) and rhu IL-4 (final concentration 250 U/ml; Strathmann Biotec, Hamburg, Germany). On day 6, a maturation cocktail consisting of TNF-α (10 ng/ml, Boehringer Ingelheim Austria, Vienna, Austria) +IL-1β (10 ng/ml; Cell Concepts, Umkirch, Germany) +IL-6 (1000 U/ml; Strathmann Biotec, Hamburg, Germany) +PGE₂ (1 Ag/ml; MinprostinR, Pharmacia and Upjohn, Erlangen, Germany) (Jonuleit, H. et al., Eur. J. Immunol.27:3135 (1997)). After 24 h the cells were harvested and cryoconserved in the culture bags.

### Example 4: Enrichment of monocytes by elutriation with PBS buffer.

Peripheral blood monocytes obtained in Example 1 were enriched as in Example 2 except that a standard elutriation buffer PBS (Bio Whittaker, Walkersville, USA) supplemented with 1% human serum albumin (Aventis-Behring, Marburg, Germany) was used instead the RPMI buffer. After elutriation the solvent of the monocyte containing fraction was removed by centrifugation and resuspended in the RPMI culture medium defined in Example 2 under sterile conditions. The DCs were then generated as in Example 3.

### Example 5: Cell morphology and phenotype

The morphology of DC was evaluated under an inverted phase contrast microscope (Leica DM IRB, Leica Mikroskopie und Systeme GmbH, Wetzlar, Germany) and was photographically documented. Cells were phenotyped using a panel of mAbs and analyzed on a FACScanR with cell questR software (Becton Dickinson) as described previously in more detail (Romani, N. et al., J. Immunol. Methods 196(2): 137 (1996)).

## Claims

1. A method for the sterile culture of blood-derived cells or bone marrow-derived cells, which method comprises enriching the cells by a separation procedure not requiring a centrifugation step and performing the enrichment or eluation step of the separation procedure with a medium close to or identical with the medium required for culture of the cells, and collecting the cells in a vessel under sterile conditions.

2. A method for the sterile culture of blood-derived cells or bone marrow-derived cells, which method comprises enriching the cells by a separation procedure not requiring a centrifugation step and performing the enrichment or eluation step of the separation procedure with a medium not suitable for culture of the cells, collecting the cells in a vessel and supplementing the enriched cell suspension in the vessel with an appropriate amount of a composition containing appropriate concentrations of all supplements required to yield a suitable culture medium.

3. The method of claim 2, which furthermore comprises a sterile cryopreservation step prior to the addition of the composition containing the supplements.

4. The method of any one of claims 1 to 3, wherein
(i) the separation procedure is elutriation, magnetic separation etc.; and/or
(ii) the blood-derived cells or bone marrow-derived cells are selected from monocytes, immature dendritic cells, CD34⁺ cells, stem cells, and any other cell contained therein; and/or
(iii) the vessel allows for sterile collection and culture of the cell suspension, sterile transfer to other culture or storage vessels, and optionally for sterile cryopreservation, preferably the vessel is a plastic bag having sealable adapters, most preferably the vessel is a Cell-Freeze bag type CF100-C3 containing a pre-attached DMSO filter which allows for closed system cryopreservation, and/or
(iv) the method further comprises culture, differentiation and/or expansion of the cells under reaction conditions and by addition of supplements required for the culture, differentiation and/or expansion of the respective cells or precursor cells.

5. The method of any one of claims 1 to 4, which is suitable for the sterile culture or generation of dendritic cells and comprises elutriation of peripheral blood mononuclear cells and culture of the monocytes obtained by the elutriation.

6. The method of claim 5, wherein the buffer for elutriation is selected from RPMI medium supplemented with autologous plasma or serum or allogenic plasma or serum (preferably with about 1% of said plasma or serum), X-Vivo 15 medium, CellGro DC Medium, AimV Culture Medium, and the like, and preferably is RPMI medium supplemented with 1% autologous human plasma or serum.

7. The method of claims 5 or 6, wherein the culture of the monocytes further comprises adding one or more differentiation factors and/or one or more maturation factors, preferably
(i) for generating stimulatory dendritic cells the differentiation factors are selected from GM-CSF, IL-4 IL-13, IFN-α, IL-3, IFN-β, TNF-α, CPG oligonucleotides and combinations of said differentiation factors, a combination of GM-CSF and IL-4, being particularly preferred; and/or
(ii) the maturation factors are selected from TNF-α, IL-1 such as IL-1β, IL-6, prostaglandins such as PGE₂, TLR ligands, CD40 crosslinking by CD40 ligand, type I interferon and combinations thereof, a maturation cocktail comprising TNF-α, IL-1β, IL-6 and PGE₂ being particularly preferred.

8. The method of any one of claims 1 to 7 which further comprises
(i) partitioning and/or cryopreservation of the cultured cells, and/or
(ii) loading the dendritic cells or the respective dendritic cell precursors with protein or lipid antigens, DNA or RNA coding for antigens, whole cells (preferably apoptotic or necrotic cells), cell fragments or cell lysates.

9. The method of any one of claims 1 to 8, wherein the cells, in particular the dendritic cells obtained by the process are suitable to be transfused or retransfused to a patient.
